Europäisches Patentamt

**European Patent Office**

**Office européen des brevets**

(11) Publication number: **0 141 616**
**A2**

# EUROPEAN PATENT APPLICATION

(21) Application number: **84307307.3**

(22) Date of filing: **24.10.84**

(51) Int. Cl.⁴: **C 12 N 5/00**, G 01 N 33/577, C 12 P 21/00

(30) Priority: **24.10.83 US 544473**

(71) Applicant: **BOARD OF REGENTS THE UNIVERSITY OF TEXAS SYSTEM, 201 West 7th Street, Austin Texas 78701 (US)**

(43) Date of publication of application: **15.05.85 Bulletin 85/20**

(72) Inventor: **Alderete, John F., 6507 Forest Grove, San Antonio Texas 78240 (US)**

(84) Designated Contracting States: **BE DE FR GB IT LU NL SE**

(74) Representative: **Clifford, Frederick Alan et al, MARKS & CLERK 57/60 Lincoln's Inn Fields, London WC2A 3LS (GB)**

(54) **Hybrid cell lines producing monoclonal antibodies directed against trichomonas vaginalis determinants.**

(57) Hybridomas secreting monoclonal antibody having specificity against antigenic determinants of Trichomonas vaginalis are provided. Diagnostic uses of the monoclonal antibody as an immunological binding reagent for the detection of Trichomonas vaginalis infection are also disclosed.

EP 0 141 616 A2

## HYBRID CELL LINES PRODUCING MONOCLONAL ANTIBODIES DIRECTED AGAINST TRICHOMONAS VAGINALIS DETERMINANTS

The present invention relates to monoclonal antibodies specific for Trichomonas vaginalis. More specifically, this invention relates to hybridomas which produce monoclonal antibodies specific for T. vaginalis antigenic determinants and their use in a diagnostic assay.

Trichomoniasis is a chronic disease of the urogenital tract caused by Trichomonas vaginalis. It is the most common of all sexually transmitted diseases and is responsible for a significant economic and emotional burden among infected individuals in this country and the world. In women, trichomonal vaginitis is characterized by inflammation of vaginal epithelium, foul-smelling discharge, and tissue cytopathology. Most men are asymptomatic, and the clinical picture of this disease remains controversial. Disease manifestations such as urethritis, prostatitis, balanoposthitis, and others, however, have been documented in infected men.

Current clinical diagnosis of trichomoniasis is tedious, time consuming, highly inadequate, and based on microscopic detection of the parasite. These limitations in diagnosis exacerbate already limited medical care in rural health clinics in our country and the world. Thus, basic research is necessary to address relevant issues such as development of sensitive, accurate assays for screening of symptomatic as well as asymptomatic patients and perhaps monitoring disease progression. The development of strategies to identify potential vaccinogen candidates is equally important and necessary.

Further, the emergence of trichomoniasis as a major sexually transmitted disease, therefore, has necessitated the need for research directed toward identification of possible virulence factors. The characterization of the surface of T. vaginalis has not been accomplished. Furthermore, the use of conventional immunological methods has failed to identify specific virulence determinants or antigens.

It therefore is highly desirable to provide monoclonal antibody to T. vaginalis antigens. Such antibodies would be important in the differential diagnosis of trichomoniasis disease in humans, in the purification of specific immunogens for subsequent use as vaccines, and studying the structure and function of immunogenic components of virulent T. vaginalis.

Antibodies are normally synthesized by lymphoid cells derived from B lymphocytes of bone marrow. The great diversity of antibody specificities is accomplished by immunoglobulin molecules having many structural features in common. Individual antibody molecules of heterogeneous binding specificity differ in their detailed amino acid sequences and even antibodies of the same specificity are usually a mixture of immunoglobulins having different amino acid sequences, although such sequences may be substantially homologous. The terms "antibody" and "immunoglobulin" are used interchangeably herein.

Individual lymphocytes produce immunoglobulin of a single amino acid sequence. Lymphocytes cannot be directly cultured to produce their specific antibody. However, Kohler, et al, Nature 256:495 (1975) demonstrated that a process of somatic cell fusion, specifically between a lymphocyte and a myeloma cell, could yield hybrid cells which grow in culture and produce a specific antibody. Myeloma cells are lymphocyte tumor cells which, depending upon the cell strain, frequently produce an antibody themselves, moreover some "non-producing" strains are known.

The hybrid resulting from somatic fusion of a lymphocyte and a myeloma cell is termed a "hybridoma" cell herein and in the art generally. In a typical fusion procedure, spleen lymphocytes from an animal immunized against a chosen antigen are fused with myeloma cells. The resulting hybridomas are then dispersed in a series of separate culture tubes or microtiter plate wells to screen for cultures producing a desired antibody. Positive cultures are further diluted to obtain colonies arising from a single cell (clones). The clones are again screened for production of the desired antibody. Antibody produced by a cloned hybridoma is termed "monoclonal" herein and in the art.

Monoclonal antibodies are highly specific, being directed against a single antigen only. Furthermore, in contrast to conventional antibody preparations which typically include different antibodies directed against different sets of determinants on the same antigen, monoclonal antibodies are directed only against a single determinant on the antigen. Monoclonal antibodies are useful to improve the selectivity and specificity of diagnostic and analytical assay methods using antigen-antibody binding. A second advantage of monoclonal antibodies is provided by the fact that they are synthesized in pure form by the hybridoma culture, uncontaminated by other immunoglobulins. Monoclonal antibodies may be prepared from supernatants of cultured hybridoma cells or from ascites induced by intraperitoneal inoculation of hybridoma cells into mice.

In accordance with the present invention, continuous hybridoma cell lines are established which elaborate and secrete highly specific and homogenous monoclonal antibodies to various antigens of Trichomonas vaginalis organisms. Specifically, hybridoma cell lines are produced which secrete monoclonal antibodies having specificity for T. vaginalis membrane protein antigens; cell surface exposed protein antigens; and certain membrane protein antigens having apparent molecular weights of 270,000; 230,000; 65,000; or 35,000.

Of the 34 hybrid clones screened which exhibited monoclonal antibodies specific for T. vaginalis, 8 hybrid lines were deposited with the American Type Culture Collection in Rockville, Maryland: hybrid cell 5bB6, C20(A3) (ATCC code - HB8379), cell F2FE10(12) (Code HB8380), cell 5bB6, C24(24) (Code HB8381), Cell 5bB6, C24(23) (Code HB8382), cell DM9 (Code HB8383), Cell DM92 (Code HB8384), cell F2FE10(C55) (Code HB8385), and cell DM86 (Code HB8386).

Further, the monoclonal antibodies according to this invention provide diagnostic reagent, useful in immunological assays for trichomoniasis infection.

The following discussion is in terms of the preferred embodiments of this invention, which represents the best mode known to the Applicant at the time of this application.

In accordance with the processes to develop the hybrid cell lines and monoclonal antibodies of this invention, test animals are stimulated for antibody production by different immunization regimens and using different antigen preparations of Trichomonas vaginalis pathogenic to humans as outlined below. For example, immunization of test animals was performed following subcutaneous inoculation of live organisms in the hind quarters of BALB/c mice or via intraperitoneal injection of either membrane material or protein-acrylamide preparations following gel electrophoresis of total protein representative of T. vaginalis and cutting of important acrylamide fractions for immunization. In all cases, Applicant has directed the preferred embodiment to immunization of mice with a heterogeneous composition of parasite materials, thereby providing a complex array of antigenic determinants.

The route and schedule of immunization of the host animal is in keeping with established and conventional techniques for antibody stimulation and production. Applicant has employed mice as a test model although it is contemplated that any mammalian subject, including human subjects or antibody-producing cells therefrom, can be manipulated to serve as the basis for production of hybrid cell lines.

After immunization, immune lymphoid cells are fused with myeloma, plasmacytoma, or hybridoma cells (hereinafter referred to collectively as myeloma cells) to generate a hybrid cell line which can be cultivated and subcultivated indefinitely, to produce large quantities of monoclonal antibodies.

For purposes of this invention, the immune lymphoid cells selected for fusion are lymphocytes and their normal differentiated progeny, taken either from lymph node tissue or spleens tissue from immunized animals. Applicant prefers to employ immune spleen cells, since they offer a more concentrated and convenient source of antibody producing cells with respect to the mouse system. The myeloma cells provide the basis for continuous propagation of the fused hybrid. Myeloma cells are tumor cells derived from plasma cells which show preference for bone marrow. Plasmacytoma cells are neoplastic cells derived from plasma cells. In particular, Applicant prefers to use lymphocyte hybridoma cells which secrete no immunoglobulin. Lymphocyte hybridoma cells are cells generated by the fusion of myeloma or plasmacytoma cells with normal differentiated lymphoid cells. Myeloma, plasmacytoma, and hybridomas can be selected to be devoid of immunoglobulin synthesis.

The particular species of animal from which the myeloma and immunized antibody producing cells are derived are not critical, in that it is possible to fuse cells of one species with another. However, it is preferred that the source of immunized antibody producing cells and myeloma be from the same species.

Generally the fusion techniques employed are according to the procedures set out by Kohler et al, _Eur. J. Immunol._ 6:11-19 (1976) and Kennett et al, _Lymphocyte Hybridomas_ - _Current Topics in Microbiology and Immunology_ 81:77-91 (1978) Springer-Verlag, New York. Fusion is generally accomplished by adding a suspension of antibody producing cells to the myeloma cells in growth medium and centrifuged to form a pellet.

The fused hybrids are next screened for antibody production specific for _T. vaginalis_ surface antigens. The membrane-specific monoclonal antibodies obtained according to preferred examples include antibodies with individual specificity for the numerous antigenic components of the membrane, including lipids, glycoprotein and protein antigenic determinants.

The hybridomas which secrete antibody specific for T. vaginalis membrane antigens are cultured to establish a continuous cell line with stable genetic coding.  These cell lines can be stored and preserved in any of a number of conventional ways, including freezing and storage under liquid nitrogen.  Frozen cell lines can be revived and cultured indefinitely with resumed synthesis and secretion of monoclonal antibodies specific for T. vaginalis antigen.  The secreted antibody is recovered from tissue culture supernatant by conventional precipitation, ion exchange, affinity chromatography, or the like.  The recovered antibody can be freeze dried and stored under refrigeration for at least several weeks without significant loss of activity.

The following examples are offered to illustrate a particular embodiment of the invention but they are not intended to limit it.

A.    Preparation of Antigen

The clinical isolate of T. vaginalis pathogenic to humans employed for generation of monoclonal antibodies crossreactive with long-term grown and fresh isolates was strain 286, which has been previously described, Alderete, Infect. Immun. 39:1041-1047, 1983.

Other strains used for the assaying of the monoclonal antibodies include a long-term grown culture, strain JH31A, purchased from the American Type Culture Collection (ATCC; Rockeville, MD) and fresh isolates obtained from Michael Spence, The Johns Hopkins University (Baltimore, MD). <u>Trichomonas vaginalis</u> was grown in a Diamond trypticase (BBL Microbiology Systems, Cockeysville, MD) yeast extract-maltose (TYM)-medium, pH 6.2, supplemented with 10% heat-inactivated horse serum (Kansas City Biologicals, Inc., Lenexa, Kansas). Organisms routinely grew to a density of 2.5 x 10 to 5 x 10" per ml as determined with an improved Neubauer counting chamber. Only motile parasites at logarithmic stage of growth were utilized. Stock cultures of all <u>T. vaginalis</u> strains were stored in liquid nitrogen in TYM-serum medium containing 10% dimethylsulfoxide (DMSO; ATCC). All strains remained virulent throughout these studies as evidenced by lesion development in mice after 9-12 days post-subcutaneous inoculation.

1. <u>Antigen used for immunization</u>

a. Preliminary studies (Alderete, <u>Infect. Immun.</u> 39:1041-1047, 1983) indicated that subcutaneous infection of the hind quarters of BALB/c mice produced antibody formation directed at highly immunogenic membrane proteins of <u>T. vaginalis</u>. For studies where spleen lymphocytes from animals challenged with live organisms were desired, the pathogenic trichomonads were grown to logarithmic phase representing $1 \times 10^6$. The organisms per ml parasites were then washed twice in sterile phosphate-buffered saline (PBS) and finally resuspended to a density of $5 \times 10^6$ per 0.5 ml volume in TYM-serum with 0.05% agar. A second injection was performed (as indicated in the next section) with organisms in TYM-serum medium followed by a third injection with <u>T. vaginalis</u> suspended in PBS.

b. For immunization with protein-acrylamide preparations, slab gel sodium dodecysulfate-polyacrylamide gel electrophoresis (SDS-PAGE) of total trichloroacetic acid (TCA)-precipitated proteins was performed using conditions and reagents previously described (Peterson and Alderete, _Infect. Immun._ 37:755, 1982; Alderete, _Infect. Immun._ 39:1041, 1983; Alderete, _Infect. Immun._ 40:284, 1983). Protein bands from one of the twenty lanes loaded with material were visualized by Coomassie Bulliant blue staining of acrylamide gels, and proteins with electrophoretic mobilities corresponding to 50,000-70,000 were excised. The gel was lyopholized, pulverized, resuspended in sterile distilled water, and this represented a protein-acrylamide antigen preparation used for immunization as outlined below. Antigen from two electrophoretic runs on a 14 x 16 cm slab acrylamide gel of 0.75 mm in thickness provided sufficient material for immunization of two mice throughout the preferred regimen. The antigen gave a final volume of 3 ml when resuspended in distilled water, and 1 ml of this material was mixed with an equal volume of Freund's complete adjuvant while the remaining two ml were mixed with Freund's incomplete adjuvant.

    c. Crude plasma membranes of _T. vaginalis_ were generated from $2 \times 10^8$ cells. The organisms were washed three times in PBS followed by resuspending the organisms in PBS containing 10 mM $MgCl_2$ and 0.5 mg/ml concanavalin A. The organisms agglutinated and were washed twice further with PBS-10 mM $MgCl_2$. Pelleted trichomonads were resuspended in 10 ml of 1 M glycerol and injected into 12 ml of 10 mM tris-hydrochloride buffer (tris-HCl), pH 7.5, containing 2 mM phenylmethyl-sulfonylfluoride (PMSF) and 1 mM $MgCl_2$. After a 10 minute incubation, the cells were homogenized further with a teflon pestle Dounce homogenizer (20 strokes) and cell lysis verified by examination of the cell preparation using darkfield and phase optics on a Zeiss IM30 microscope. The homogenate was then layered on a two-step gradient consisting of 8 ml of 0.5 M mannitol over 4 ml of 0.58 M sucrose. The gradient was centrifuged at 250 xg for 30 minutes and

pellet represented crude membranes used for immunization protocols. Electrophoretic analysis of crude membrane material confirmed the presence of immunogenic proteins and glycoproteins previously identified as residing on T. vaginalis membranes (Alderete, Infect. Immun. 40:284, 1983). Protein values were determined by the Bradford technique (Bradford, Anal. Biochem. 72:248, 1976).

B.    Immunization Schedule for Hybridoma Production

Different immunization protocols were used depending on antigen employed for generating immune spleen cells. Monoclonal antibodies illustrated in this embodiment are from three separate hybridization experiments, each representing the antigen described above. Spleen cells from two mice were used for each hybridization. Six to eight week old BALB/CJ female mice (Jackson Laboratories, Bar Harbor, Maine) were employed for these studies.

1.    Live organisms

Mice were infected in the hind quarters by subcutaneous injection of 0.5 ml containing no less than $5 \times 10^6$ organisms per site. A second booster inoculation was performed at day 20 and a final challenge was given at day 30. Spleen cells were asceptically removed for use in hybridoma production from the immunized mice three days after the last inoculation with live organisms. This regimen allowed for the production of hightitered serum antibody levels as assayed by colorimetric (ELISA) and radioimmunoprecipitation-electrophoretic (RIP) techniques.

2.    Protein-acrylamide and crude membrane antigen

Each of two mice received an initial intraperitoneal injection with protein-acrylamide antigen in Freund's complete adjuvant followed by two subsequent booster injections at day 14 and day 28 of protein-acrylamide in Freund's incomplete adjuvant.

Similarly, each of two mice were given intraperitoneal injections with 1 ml of a 1:1 mixture of crude membrane (2 mg protein/ml) and Freund's complete adjuvant. Two booster inoculums also given intraperitoneally with identical protein levels in Freund's incomplete adjuvant at days 14 and 28 after the initial injection. Spleens from each of both mice were removed for use in hybridoma production from the immunized mice three days after the last injection of antigen.

C.    Construction of Hybridomas

Hybridomas were produced by fusing spleen cells from the immunized mice with murine SP2/0-Ag14 hybridoma cells (SP2/0 hereinafter) using a modification of the basic procedure of Oi and Herzenberg, Immunoglobulin-Producing Hybrid Cell Lines, In Selected Methods in Cellular Immunology, B.B. Mishell and S.M. Shiigi, eds., pp. 351-371, W.H. Freeman and Co., 1980, San Francisco. Suitable cell lines were obtained from Ed Hayes, Duke University and are as originally set forth by Schulman et al, Nature 276:269-270 (1978). The SP2/0 hybridoma cell line is a hybrid cell line derived from SP2/HGLK formed as a hybrid between a BALB/c spleen cell and the myeloma cell lines X63-Ag8. This cell line synthesizes no immunoglobulin chains, lacks the enzyme hypoxanthine guanine phosphori-bosyl-transferase (HGPRT), is resistant to 8-azaguanine, and dies in the presence of Littlefield's hypoxanthine-aminopterin-thymidine (HAT) selection medium. SP2/0 cells were grown in Dulbecco's Modified Eagle's Medium (DMEM) (Microbiological Associates, Walkersville, MD) supplemented with 15% (vol/vol) heat-inactivated fetal calf serum (Microbiological Associates), 2 mM L-glutamine, and 50 units/ml penicillin and 50 g/ml streptomycin. SP2/0 cells were grown in this medium containing 8-azaguanine (20 µg/ml) immediately prior to use in hybridization experiments to ensure that no HGPRT-positive revertants were present in the cell culture.

Spleens were removed aseptically from immunized mice and teased apart gently with forceps to prepare a single cell suspension in DMEM-1 mM Hepes buffer (Microbiological Associates). SP2/0 cells were harvested in the logarithmic phase of growth and both cell types were collected by centrifugation at 270 x g for 10 minutes at 8°C and washed three times with DMEM. Total cell numbers were determined with a Neubauer Counting hemocytometer and viability was measured by trypan blue exclusion.

Approximately $10^8$ spleen cells were mixed together with SP2/0 cells in a 50 ml conical tube at a ratio of 7 viable spleen cells per viable SP2/0 cell and the resultant cell suspension was collected in a pellet by centrifugation at 270 x g for 10 minutes. The supernatant medium was removed and the tube containing the cell pellet was placed in a 37°C water bath for 1 minute. A 1.0 ml portion of a warm (37°C) 50% (wt/vol) solution of polyethylene glycol (PEG 1000; ATCC) in DMEM per 1-2 x $10^8$ spleen cells was added to the cell pellet with gentle stirring over a 1 minute period. The suspension was stirred an additional minute. Then, 1 ml (per 1-2 x $10^8$ spleen cells) of DMEM was added over an additional 1 minute period and this step repeated once more. Finally, 7 ml (per 1-2 x $10^8$ spleen cells) DMEM with 20% fetal bovine serum (tested for hybridoma growth, Microbiological Associates) was added over 2-3 minutes. The cells were then centrifuged at 400 x g for 10 minutes and cells resuspended to 2-4 x $10^6$ per ml in HY medium (Kennett et al, Lymphocyte Hybridomas - Current Topics in Microbiology and Immunology, Vol. 81, pp. 77-91 (1978) Springerverlag, New York) and dispensed in 50 µl portions containing 2-4 x $10^5$ cells into each well of 96-well plates (Costar Plastics, Vineland, New Jersey), which were then incubated at 37°C in a humidified incubator containing a 7% $CO_2$ atmosphere.

On day one, a 50 µl portion of a two-fold concentration of Littlefield's HAT selection medium specified in Littlefield et al, Science 145:709-710 (1964) was added to each well. An additional 50 µl portion of HAT was added to each well on day four.

The unfused SP2/0 cells died in HAT within 24-48 hours. Cell growth in HAT medium is indicative of successful hybridization. Hybrid clones selected in HAT were usually observed by day six. After day six, all wells were fed (HT)-glycine medium, which comprises HY medium containing $1.6 \times 10^{-4}$M hypoxanthine, and $3 \times 10^{-6}$M glycine. Wells which contained growing clones were assayed to detect monoclonal antibodies directed at T. vaginalis surface antigens and cells where supernatants were positive for antibody toward parasites were transferred from these individual wells into a respective well of a 24-well tissue culture plate (Costar Plastics). Hybrid clones were maintained in HY medium without feeder layers and sequentially expanded to grow in T75 (750mm$^2$ flasks; Costar) for freezing of cell lines in 90% Fetal bovine serum (Microbiological Associates) - 10% DMSO (ATCC) in liquid nitrogen.

D.    Characterization of Monoclonal Antibody

Analysis of Anti-T. vaginalis Membrane
Antigen Activity in Hybridoma Supernatants

Screening of hybrid clone culture supernatants for the presence of monoclonal antibodies directed against T. vaginalis membrane antigens was performed using an ELISA technique. Live trichomonas in logarithmic phase of growth were washed well in PBS and 50 μl representing 1.25 x $10^5$ organisms were plated onto individual Immulon II strip wells (Dynatech, Alexandria, VA). The cells were allowed to dry at 37°C followed by fixation with 100% ethanol. Antigen-coated wells remained stable over a period of 6 months and were stored at 4°C until use.

Antigen-coated wells were washed three times with pH 7.2 phosphate buffered saline (PBS). PBS (300 μl) containing 1% (wt/vol) bovine serum albumin (BSA) was incubated in each microtiter well for 1 hour at room temperature to saturate nonspecific protein binding sites in the plastic well. This solution was then removed by aspiration, the wells were washed three times with PBS, 100 μl of hybrid clone culture supernatant was added to the well and the microtiter plate was incubated at 37°C for 120 minutes. Positive control wells contained mouse serum obtained from the same mouse whose spleen was employed for hybridization.

Supernatant fluid was then removed by aspiration, the microtiter wells washed three times with PBS, and alkaline phosphatase-conjugated goat anti-mouse immunoglobulins (Cappel Laboratories, Cochranville, Pennsylvania), prepared by the method of Voller et al, Bull. WHO 53:55-65, 1976, and diluted 1/500 in PBS, was then added to a final volume of 200 μl in each microtiter well. The microtiter plates were incubated at 37°C for one hour after which the conjugated antisera was removed by aspiration, the wells washed three times with PBS, and 300 μl of enzyme substrate [p-nitrophenyl phosphate (Sigma); 1 mg/ml in 10% (vol/vol) diethanolamine buffer (pH 9.8) containing 1 mM $MgCl_2$] was added to each well. Thirty minutes later, the absorbance of the solution in each well was determined spectrophotometrically at 405 nm using a Dynatech Micro-ELISA reader (Dynatech, Alexandria, MD). Microtiter wells in which the absorbance was at least two-fold greater than background levels of absorbance obtained with antigen-free control wells were scored as positive for the presence of antibodies directed against T. vaginalis membrane antigens.

Of approximately 500 hybrid clones screened, 28 stable hybrids demonstrated antibody reactive against T. vaginalis antigen determinants. A listing of hybrids detected is shown in Table 1.

Isotypic Analysis

Cultures positive for antibodies against T. vaginalis were next tested to identify the mouse antibody isotype, using standard immunological assays employing rabbit anti-mouse subclass antibody reagents and peroxidase-conjugated affinity purified goat anti-rabbit IgG (H&L) for use in standard ELISA (Mouse immunoglobulin subtype identification kit, Boehringen Mannheim Biochemicals, Indianapolis, IN).

For subtyping, 50 µl hybridoma supernatant was incubated with T. vaginalis whole cell antigen as above after coating of nonspecific sites with BSA. After 2 hours at 37°C, the wells were washed four times with PBS. Then 50 µl of each subclass specific immunoglobulin were added to duplicate wells. Normal rabbit serum served as a negative control. After a further incubation for 2 hours and washing four times with PBS, 50 µl peroxidase-labelled goat anti-rabbit IgG antibody was added to each well. The reaction was allowed to proceed for 60 minutes and wells washed another four times with PBS. Finally, 100 µl freshly prepared peroxide reagent was added to each well, and the reaction terminated after 30 minutes. Color was scanned using a Dynatech spectrophotometer at 415 nm.

Isotypic analysis for each hybrid is listed in Table 1.

Analysis of Anti-T. vaginalis Membrane
Protein Activity in Hybridoma Supernatants

Culture supernatant fluids from hybrid clones which scored positive in the ELISA test were assayed by a radioimmunoprecipitation method (Alderete, Infect. Immun. 39:1041, 1983 and Alderete, Infect. Immun. 40:284, 1983) for the presence of monoclonal antibodies directed against trichomonal membrane proteins.

Membrane proteins of T. vaginalis were radioiodinated by the method of Alderete, Infect. Immun. 40:284, 1983. Radioiodinated cells were extensively washed with PBS and pelleted organisms resuspended with 200 µl of NET (150 mM NaCl, 5 mM EDTA, 50 mM Tris-hydrochloride, pH 7.2) buffer containing 1 mM PMSF (Sigma) and placed in a 37°C water bath for 10 minutes. Twenty-five microliters of 10% Zwitterionic 3-12 (Z3-12) detergent (Calbiochem-Behring Corp., La Jolla, Calif.) was then added, and the mixture was gently homogenized until the trichomonads were solubilized. An additional 200 µl of NET buffer was added, and the detergent extract was centrifuged over a 5% sucrose bed for 30 minutes at 100,000 x g with a Beckman SW50.1 rotor. Greater than 80% of the initial radioactivity was routinely recovered in the supernatant.

Before mixing with antibody, the Z3-12 extract was preadsorbed with 100 µl of 10% (vol/vol) Formalin-fixed, protein A-bearing S. aureus to remove nonspecific binding proteins. Then 100 µl of the adsorbed solubilized trichomonal proteins was mixed with 50 µl of hybridoma supernatant and incubated overnight at 4°C. Finally, 100 µl of 10% (vol/vol) fixed protein A-bearing S. aureus was added, and incubation was continued at room temperature for a further 2 hours.  The protein A-bearing S. aureus-adsorbed immune complexes were then sedimented at 10,000 x g for 4 minutes and washed three times in NET-0.05% Z3-12 buffer.  Radiolabeled antigen-antibody complexes were then solubilized in electrophoresis dissolving buffer.  The samples were suspended and boiled for three minutes, and protein A-bearing S. aureus cells were removed by centrifugation.  The supernatants containing parasite protein antigens were finally loaded on SDS-polyacrylamide slab gels.  Electrophoresis was performed as described (Alderete, Infect. Immun. 39:1041, 1983), and gels were fixed and processed for fluorography.  The protein A-bearing S. aureus cells were sequentially washed in 0.5% Z3-12 and 0.05% Z3-12 in NET buffer just before use.  The formaldehyde-fixed protein A-bearing S. aureus organisms employed in these studies were grown and prepared as described elsewhere (Alderete and Baseman, Infect. Immun. 26:1048, 1979).  Equally important was immunoprecipitation of trichomonal proteins biosynthesized and radiolabelled with [$^{35}$S]-methionine with molecular weights identical to those obtained with radio-iodinated organisms.  Hence, these antigens are parasite derived membrane proteins.

Table 1. Monoclonal Antibodies Directed Against T. vaginalis Membrane Protein Antigens.

| Fusion[1] Type | Hybrid Clone | Reactivity with[2] other strains | Reacts w/[3] Shed Ag | FITC[4] | ELISA reactivity | Membrane Antigen ($^{35}$S/$^{125}$I-Protein) | | Immuno-globulin type |
|---|---|---|---|---|---|---|---|---|
| A | F2FE10,(C55) | 7/7 | + | - | + | + | + 65K | $IgG_1,\lambda$ |
| A | F2FE10,(18) | 7/7 | + | - | + | + | + 65K | $IgG_1,\lambda$ |
| A | 5bB6,C24,(24) | 7/7 | + | + | + | + | + 270K | $IgG_2a,K$ |
| A | 5bB6,C24,(23/12) | 7/7 | + | + | + | + | + 65K | $IgG_2a,K$ |
| A | 5bB6,C20(A3) | 7/7 | + | + | + | + | + 230K | $IgG_2a,K$ |
| A | F11D3,C10 | 7/7 | + | - | + | + | - 35K | $IgM,K$ |
| B | DM9 | 7/7 | + | + | + | + | + 270K | $IgG_1,K$ |
| B | DM86 | 7/7 | + | + | + | + | + 270K | $IgG_1,K$ |
| B | DM92 | 7/7 | + | + | + | + | + 270K | $IgG_1,K$ |
| C | Acryl 65,1 | 7/7 | + | - | + | + | + 65K | $IgM$ |
| C | Acryl 65,5 | 7/7 | + | - | + | + | + 65K | $IgG_1$ |
| C | Acryl 65,7 | 7/7 | + | - | + | + | + 65K | $IgG_1,K$ |
| C | Acryl 65,11 | 7/7 | + | - | + | + | + 65K | $IgG_1,\lambda$ |
| C | Acryl 65,15 | 7/7 | + | - | + | + | + 65K | $IgG_1,K$ |
| C | Acryl 65,20 | 7/7 | + | - | + | + | + 65K | $IgG_1,K$ |
| C | Acryl 65,21 | 7/7 | + | - | + | + | + 65K | $IgG_1$ |
| C | Acryl 65,22 | 7/7 | + | - | + | + | + 65K | $IgG_1$ |
| C | Acryl 65,23 | 7/7 | + | - | + | + | + 65K | $IgG_1,K$ |

TABLE 1

| Fusion[1] Type | Hybrid Clone | Reactivity with[2] other strains | Reacts w/[3] Shed Ag | FITC[4] | ELISA reactivity | Membrane Antigen ($^{35}S/^{125}I$-Protein) | | Immuno-globulin type |
|---|---|---|---|---|---|---|---|---|
| C | Acryl 65,24 | 7/7 | + | - | + | + | + 65K | IgM,K |
| C | Acryl 65,25 | 7/7 | + | - | + | + | + 65K | $IgG_{2_a}$,K |
| C | Acryl 65,26 | 7/7 | + | - | + | + | + 65K | $IgG_1$,K |
| C | Acryl 65,29 | 7/7 | + | - | + | + | + 65K | $IgG_{2_a}$,λ |
| C | Acryl 65,35 | 7/7 | + | - | + | + | + 65K | - |
| C | Acryl 65,36 | 7/7 | + | - | + | + | + 65K | $IgG_1$,K |
| C | Acryl 65,38 | 7/7 | + | - | + | + | + 65K | $IgG_1$,K |
| C | Acryl 65,40 | 7/7 | + | - | + | + | + 65K | $IgG_1$,K |
| C | Acryl 65,41 | 7/7 | + | - | + | + | + 65K | $IgG_1$,K |
| C | Acryl 65,47 | 7/7 | + | - | + | + | + 65K | $IgG_1$,K |

[1]Fusion type: A, B and C refer to hybridoma cells obtained from fusion of spleen cells from mice immunized with live organisms, membrane preparations, or protein-acrylamide antigen, respectively, as described in the application.

[2]Reactivity detected in seven of seven strains representing two long-term grown cultures and five fresh isolates as determined by ELISA.

[3]ELISA reactivity using ascites monoclonal antibody as the attractor molecule on polyvinylchloride microtiter plates for binding of T. vaginalis molecules shed during growth and multiplication. ND, not done

[4]Fluorescein isothiocyanate (FITC) indirect immunofluorescence using monoclonal antibody incubated with live parasites.

TABLE 1 CONTINUED

-19-

A total of 28 monoclonal antibodies (see Table 1) obtained from 3 representative hybridization experiments using different antigens were shown to be directed at T. vaginalis membrane proteins previously shown to be highly immunogenic in mice infected with live parasites (Alderete, Infect. Immun. 34:1041, 1983). Four different T. vaginalis membrane proteins with apparent molecular weights of 270,000 (270K), 230K, 65K, and 35K were recognized by one or more of these monoclonal antibodies. The 65K and 35K proteins are quantitatively dominant in gel profiles of total TCA-precipitated protein material while all of these molecules are quantitatively enriched in crude membrane preparations.

Monoclonal antibodies 5bB6C24(24), DM9, DM10, and DM89 were specific for a highly immunogenic membrane protein of 270K in molecular weight. Monoclonal antibody 5bB6C20(A3) was directed against a 230K protein. Monoclonal antibodies F2FE10(C55), F2FE10(18), 5bB6C24(23), and nineteen monoclonals from fusion type C were against a membrane protein of 65K in size. It is important to note that the 65K protein band of TCA-precipitated trichomonal proteins is composed of up to 30 proteins based on 2-dimensional electrophoretic analysis. The exact identity of the 65K proteins, separable by isoelectric focusing reactive with the respective monoclonal antibody is unknown.

However, it is anticipated that the major immunogens of 65K molecular weight proteins are reactive with the monoclonals listed in Table 1 (fusion type C).

## Analysis of Anti-T. vaginalis Cell Surface-Exposed Protein Activity

The twenty-eight monoclonal antibodies listed in Table 1 that were shown to be directed against T. vaginalis membrane proteins were identified in radioimmunoprecipitation assays using solubilized membrane proteins as antigen. In order to determine if any of these monoclonal antibodies were directed against cell surface-exposed portions of these outer membrane proteins, hybrid clone supernatants were used in indirect immunofluorescence assays. Adsorption of hybrid clone culture supernatants with intact cells was accomplished by incubating 200-500 µl of hybrid clone culture supernatant with $1 \times 10^5$ intact, washed trichomonads for 30 minutes at 37°C. Only three monoclonal antibodies from fusion "A", F1iD3C10, F2FE10(C55) and F2FE10(18), and all monoclonal antibodies from fusion "C" could not be detected by fluorescence microscopy. The remaining monoclonal antibodies were readily detected by strong fluorescence of parasites, and hence, indicative of recognizing a cell surface exposed protein antigen. The proteins recognized by these antibodies are highly immunogenic membrane antigens, and epitopes essential for recognition by these antibodies are exposed on live, motile organisms.

## Strain Distribution of the Antigenic Determinant

It was of interest to determine if the antigenic determinant recognized by the monoclonal antibodies were unique to the immunizing T. vaginalis strain or whether this antigenic determinant might be found in other pathogenic human trichomonal strains. Accordingly, six different clinical isolates of T. vaginalis (strains 286, JH31A, JHHZH, JHHR, JHHEL, and JHHW) collected over a two year period were examined for the presence of the antigenic determinant recognized by monoclonal antibodies. Small (200 μl) portions of respective culture supernatants were employed in the standard ELISA assay used to detect monoclonal antibody activity directed against membrane proteins. All monoclonal antibodies described in Table 1 detected all strains to the same extent as the homologous strain employed as the immunization agent, strain 286. Importantly, strains 286 and JH31A were cultivated in-vitro for several years and thus represented long-term grown cultures while the other strains were fresh isolates grown for no longer than 3-4 days in Applicant's laboratory prior to testing.

E.    Immunodiagnosis of Trichomonal Vaginitis

The hybridoma cell lines and the monoclonal antibodies produced therefrom described in this application are useful for detection of T. vaginalis antigen in vaginal swabs of infected individuals. As such, they represent candidates for development of an immunodiagnostic test for differentiation of trichomonal vaginitis from similar symptomology caused by other etiologic agents.

EXAMPLE I

A subset of monoclonal antibodies representative of the four membrane proteins recognized by the antibodies by radioimmunoprecipitation-electrophoretic techniques just described were evaluated for detection of trichomonal antigen in swabs from four different infected individuals. The monoclonal antibody test reagents were derived from the respective hybrid clones: F2FE10(C55), F2FE10(C18), 5bB6C20(A3), and 5bB6,C24(24).

The diagnostic test samples consisting of vaginal material from infected humans were provided on cotton swabs placed in sterile screw-capped 15 x 125 mm tubes and were received from Dr. Michael Spence, The Johns Hopkins University. Upon arrival in our laboratory, the swabs were immersed in 1 ml sterile PBS with 1 mM PMSF and vortexed. Soluble material was clarified from particulate debris by centrifugation at 10,000 x g for 10 minutes, and supernatant containing possible aqueous soluble parasite material was frozen at -70°C until use. Pellet was dissolved in 0.05% Zwittengent 3-12 detergent (Calbiochem. Behring, Inc., La Jolla, CA) re-clarified by centrifugation, and detergent-soluble material also frozen (-70°C) until use.

Detection of T. vaginalis proteins with monoclonal antibody reagents was accomplished by ELISA using respective antibody obtained from ascites fluid as the attractor molecule adsorbed to microtiter well plates by the Method of Voller (described supra). One microgram (1µ g) ascites protein was added to individual wells of polyvinylchloride Immunlon II microtiter plates (Dynatech) and incubation continued at 37°C overnight. Prior to use in ELISA, nonspecific binding sites were blocked with a 2 hour incubation with PBS-BSA as before. To individual wells, 100 µl of aqueous or detergent-soluble infected human swab material was added and plates placed at 37°C for 2 hours. Plates were then washed three times with PBS, and 50 µl of a polyclonal rabbit antiserum to strain 286 added. After an additional 60 minutes incubation at 37°C, wells were washed three times with PBS and alkaline phosphatase goat anti-rabbit antibody added. Upon incubation at 37°C for 60 minutes, wells were washed again three times with PBS, substrate added, and wells placed in 37°C incubated for 60 minutes further. Color was recorded at 405 nm using a Dynatech microtiter well scanner. Control material represented a similar preparation and swab obtained from a normal individual with no history of trichomoniasis or an individual with a vaginitis of a different etiology.

Each of the four tested monoclonal antibody types reacted positively with each of the sample infected materials. None of the tested monoclonal antibody types reacted significantly with the control sample material from uninfected individuals.

EXAMPLE 2

An alternative method for testing the efficacy of representative monoclonal antibodies for detecting T. vaginalis membrane proteins in body fluids of infected individuals was performed. Five to 7 ml of vaginal wash obtained by injection of 10 ml sterile PBS into the vagina of control and infected volunteer females was obtained from Michael Spence, Johns Hopkins School of Medicine. The wash was clarified by centrifugation, and supernatant concentrated by lyophilization. Material was re-dissolved into 500 µl volumes using carbonate buffer (Voller), and 50 µl aliquots plated into individual wells of microtiter well plates (Immulon II, Dynatech). Polyclonal mouse antiserum (50 µl) against T. vaginalis 286 and each of the individual monoclonal antibodies F2FE10(C55); F2FE10(C18); 5bB6C20(A3) and 5bB6C24(24) were then added to respective wells coated with concentrated vaginal wash material. After a 60 minute incubation, the wells were washed three times with PBS, and alkaline phosphatase goat anti-mouse antibody added to each well. After a 60 minutes incubation at 37°C, plates were washed, and substrate added. Reactivity was recorded at 405 nm using a Dynatech spectrophotometric scanner. Each of the four tested monoclonal antibody types were reactive with each of the wash samples taken from infected individuals. None of the monoclonal antibodies reacted to a significant degree with wash samples from uninfected individuals.

EXAMPLE 3

Because testing of all monoclonal antibodies generated from the successful hybridomas could not be extensively evaluated using human materials, the ability of the monoclonal antibodies to detect T. vaginalis membrane proteins shed during growth and multiplication was evaluated. Since all trichomonal strains shed membrane proteins during the course of logarithmic growth, these membrane proteins might be present in body fluids (vaginal wash, mucous, swab). An ELISA, therefore, was used to measure parasite antigen detected by the monoclonal antibodies. Monoclonal antibodies (1μ g) from ascites fluid were adsorbed onto microtiter plate wells (Immulon II, Dynatech) as before, and 100 μl of culture supernatant from logarithmically-growing wells added to each well. All wells were pretreated with PBS-BSA to coat nonspecific binding sites. After 2 hours at 37°C, rabbit anti-serum against strain 286 was added and wells placed in a 37°C incubator for 60 minutes. The wells were then washed again with PBS, and added alkaline-phosphatase goat anti-mouse allowed to incubate at 37°C for 60 minutes. After washing of the wells with PBS, substrate was added, and color, indicative of a positive reaction, was recorded at 405 nm. Controls represented monoclonal antibody generated against protein antigens of M. pneumoniae as well as normal rabbit serum. Also, sterile un-inoculated medium was employed to show the parasite origin of detected materials. Results are tabulated in Table 1, (reacts with Shed Ag) for each of the monoclonal antibody types.

F. <u>Utility</u>

The hybridoma cell lines and the monoclonal antibodies produced therefrom described in this application are useful in the purification and characterization of specific antigenic and immunogenic components presented by <u>Trichomonas vaginalis</u> organisms. Moreover, the monoclonal antibodies produced from a given hybridoma line are homogeneous in antigenic recognition and thereby are useful for subsequent affinity chromatography-based purification of trichomonal membrane antigens.

Furthermore, the availability of different monoclonal antibodies directed against one or more antigenic determinants of the same membrane antigen of <u>T. vaginalis</u> is useful in studying the structure and function of membrane components. Similarly, these same monoclonal antibodies are valuable in the idiotypic analysis of antibody response to cell surface structure of a pathogenic microorganism.

Ultimately, the availability of monoclonal antibodies directed against selected <u>T. vaginalis</u> membrane antigens, in particular cell surface-exposed membrane proteins, will facilitate studies on the vaccinogenic potential of these proteins.

The foregoing description of the invention has been directed to particular embodiments for purposes of explanation and illustration. It will be apparent, however, to those skilled in the art that many modifications and changes in the processes of preparing and implementing the described embodiments may be made without departing from the essence of the invention.

For example, it is contemplated that hybridoma cell lines may be developed fusing human myeloma cells and human lymphocytes primed to membrane antigens of T. vaginalis.

In another example, monoclonal antibodies can be developed which are specific for individual T. vaginalis strains other than strain 286 used in these hybridization experiment. Also, hybridoma cells can be constructed from the differentiated lymphoid cells of mice immunized by alternate routes and methods. Similarly, other mouse strains can be used to produce hybridoma cells elaborating similar sets of monoclonal antibodies suitable to the purposes described herein. Moreover, monoclonal antibodies need not be limited against protein components of the membrane. Development of hybridoma producing antibody against carbohydrate moieties of the membrane glycoproteins or unique lipids found only in trichomonads can be developed according to the processes of this invention. Such antibodies would be useful in the purification, isolation, and structural determination of the same or other molecules of the membrane of Trichomonas vaginalis. These and other modifications and uses of the depicted embodiment, as well as other embodiments of the invention, will be apparent to those skilled in the art. It is Applicant's intention in the following claims to cover all equivalent modifications and variations as fall within the scope of the invention.

CLAIMS:

1. A composition of matter which is characterized as a continuous hybrid cell line that produces monoclonal antibody against antigenic determinants of Trichomonas vaginalis, which cell line is formed as fusion between a myeloma cell and a lymphocyte immunized against Trichomonas vaginalis antigen.

2. The composition of matter of claim 1 wherein the hybrid cell line is a cell hybrid of a mouse lymphocyte immunized against Trichomonas vaginalis antigen, fused to a mouse myeloma cell.

3. The composition of matter of claim 1 wherein the continuous hybrid cell line is a cell hybrid of a BALB/c mouse immune spleen cell immunized against Trichomonas vaginalis antigen, fused to a mouse myeloma cell.

4. The composition of matter of claim 1 wherein the hybrid cell line is a cell hybrid of a mouse lymphocyte immunized against Trichomonas vaginalis antigen, fused to a SP2/0 hybridoma cell.

5. The composition of matter of claim 1 wherein the hybrid cell line consists essentially of hybridoma clone ATCC deposit HB8379, HB8380, HB8381, HB8382, HB8383, HB8384, HB8385, or HB8386.

6.    The composition of matter of claim 1 wherein the Trichomonas vaginalis antigen is an membrane protein antigen.

7.    The composition of matter of claim 1 wherein the Trichomonas vaginalis antigen is a cell surface exposed protein antigen.

8.    The composition of matter of claim 1 wherein the Trichomonas vaginalis antigen is a membrane protein antigen with an apparent molecular weight of 270,000; 230,000; 65,000; or 35,000.

9.    The composition of matter of claim 1 wherein the Trichomonas vaginalis antigen is a membrane protein antigen with an apparent molecular weight of 65,000.

10.   A composition of matter which is characterized as monoclonal antibody specific for an antigenic determinant of Trichomonas vaginalis.

11.   The composition of matter of claim 10 wherein the monoclonal antibody is specific for a membrane protein antigen of Trichomonas vaginalis.

12.   The composition of matter of claim 10 wherein the monoclonal antibody is a cell surface exposed protein antigen of Trichomonas vaginalis.

13. The composition of matter of claim 10 wherein the monoclonal antibody is membrane protein antigen with an apparent molecular weight of 270,000; 230,000; 65,000; or 35,000.

14. The composition of matter of claim 10 wherein the monoclonal antibody is membrane protein antigen with an apparent molecular weight of 65,000.

15. The composition of matter of claim 10 wherein the monoclonal antibody is produced from hybridoma clone ATCC deposit HB8379, HB8380, HB8381, HB8382, HB8383, Hb8384, HB8385, or HB8386.

16. A method of diagnosing Trichomonas vaginalis infection in a host which is characterized as comprising:

   contacting a physiological sample from the host together with a composition of matter comprising monoclonal antibody specific for an antigenic determinant of Trichomonas vaginalis;

   measuring the immunological binding reactivity between antigenic material of the physiological sample and the monoclonal antibodies.

17. The method according to claim 16 wherein the physiological sample is vaginal wash material.

18. The method according to claim 16 wherein the physiological sample is vaginal tissue sample.

0141616

19. The method according to claim 16 wherein the monoclonal antibody is specific for a membrane protein antigen of <u>Trichomonas vaginalis</u>.

20. The method according to claim 16 wherein the monoclonal antibody is specific for a cell surface exposed protein antigen of <u>Trichomonas vaginalis</u>.

21. The method according to claim 16 wherein the monoclonal antibody is specific for a membrane protein antigen with an apparent molecular weight of 270,000; 230,000; 65,000; or 35,000.

22. The method according to claim 16 wherein the monoclonal antibody is specific for a membrane protein antigen with an apparent molecular weight of 65,000.